# EUROPEAN PATENT APPLICATION

(11) **EP 2 932 966 A1**
(43) Date of publication of application: **21.10.2015**
(21) Application number: 14164980.6
(22) Date of filing: 16.04.2014
(51) Int. Cl.: A61K 31/00, A61K 31/145, A61K 31/381, A61K 31/4164, A61K 31/4245, A61K 31/55, A61P 11/00, A61P 11/06

(54) **Gamma secretase inhibitors for treating respiratory diseases**

(71) Applicant: Novartis AG, 4056 Basel (CH)
(72) Inventor: The designation of the inventor has not yet been filed
(74) Representative: Campbell, Lachlan Clive

(57) **Abstract**

The invention is in the field of treatment of respiratory diseases using a gamma secretase inhibitor. In particular, it relates to the treatment of cystic fibrosis (CF), primary ciliary dyskinesia, chronic bronchitis, chronic obstructive pulmonary disease (COPD), asthma or respiratory tract infections using a gamma secretase inhibitor.

## Description

### Field of the invention

The invention is in the field of treatment of respiratory diseases using a gamma secretase inhibitor. In particular, it relates to the treatment of cystic fibrosis (CF), primary ciliary dyskinesia, chronic bronchitis, chronic obstructive pulmonary disease (COPD), asthma or respiratory tract infections using a gamma secretase inhibitor.

### Background of the Invention

The lung epithelium has evolved to serve a number of functions ranging from gas exchange in the alveolus to the regulation of mucus clearance in the larger conducting airways. The heterogeneous mix of epithelial cell types enables these functions at the different levels of the airway. Airway epithelial cells include mucin producing and secreting goblet cells, which provide a mucus gel for the multi-ciliated cells to propel out of the airways, surfactant producing type 2 pneumocytes, which maintain alveolar patency with type 1 pneumocytes enabling gas exchange, and basal cells, the progenitor of the overlying epithelium (Rackley, C.R. & Stripp, B.R., The Journal of Clinical Investigation 122, 2724-2730 (2012)).

The protective role of the airway epithelium depends on a highly effective defence provided by airway mucus. Excessive mucus or impaired mucus clearance contributes to the pathogenesis of many common and less common airways diseases. The accumulation of mucus results from some combination of overproduction and decreased clearance and persistent accumulation can lead to infection, inflammation and suitable conditions for microbial growth (Fahy J.V. & Dickey B.F., The New England Journal of Medicine, 363, 2233-2247 (2010)).

Mucus hyper-secretion has been suggested to be an important pathological feature of chronic obstructive pulmonary disease (COPD) (Prescott, E.I. et al., 158, 6456 (1996)), asthma (Aikawa, T. et al., T. Chest, 101, 916 (1992)) and cystic fibrosis (Boucher, R.C. Adv. Drug Deliv. Rev., 54, 1359 (2002)). Substantial epidemiological studies have demonstrated an association between mucus hyper-secretion and increased frequency and duration of respiratory infection, hospitalization, increased levels of morbidity and mortality. It is presently clearly recognised that a hallmark of many airways diseases is an overabundance of goblet cells, referred to as mucus hypersecretory phenotype. Mucus and the cells that produce it form an integral part of the mucociliary clearance apparatus whose co-ordinated function is considered essential to the protection of the airways from irritant/infectious insult. In healthy airways, secreted mucin is cleared by mucociliary clearance, infrequently assisted by conscious coughing, and does not therefore accumulate. In contrast, plugging of the small airways with mucus together with chronic cough and phlegm production are common manifestations in respiratory diseases.

Small airway plugging with mucus is a major contributing factor to fatal asthma, especially in younger patients (Kuyper, L.M. et al.; Am. J. Med., 115, (2003)), and is associated with goblet cell hyperplasia (Aikawa, T. et al., T. Chest, 101, 916 (1992)). In addition, 20% of asthmatics report chronic cough and phlegm production (Cerveri, S. et al.; Eur Respir J 22: 413-417 (2003)) and highlights the poorly controlled population (de Marco R. et al.; Am J Respir Crit Care Med 175, 32-39 (2007)). These patients are also those with significantly more upper and lower respiratory symptoms (Timonen K.L. et al.; Eur Respir J. Mar 19, 479-86 (2002)). Importantly, chronic mucus hypersecretion has also been identified as a significant marker of an enhanced decline in lung function in asthmatics (Lange P. et al.; N Engl J Med. 339, 1194-2000 (1998); Ulrik C.S. & Lange P. Am J Respir Crit Care Med. 150, 629-34 (1994)).

Goblet cells, which in healthy subjects are restricted to large airways, have been shown to be increased in numbers in small airways of patients affected by COPD and other respiratory diseases (Hogg J.C. Novartis Foundation Symposium, 234, 4 (2001). Whether goblet cell numbers increase as a consequence of progenitor cell proliferation (hyperplasia), non-mitotic differentiation (metaplasia) or inhibition of necrotic/apoptotic processes or whether one process predominates over the other is still poorly understood. In the past decades, scientists have been elucidating molecules and pathways that regulate cell fate decisions. Most of these molecules operate in multiple tissues, at different stages of development or disease stage. Notch signaling is an evolutionarily conserved pathway that regulates many cell-fate decisions during development (Fortini, M.E., Dev Cell 16, 633-647 (2009)). Notch signaling in regulating cell fate decisions during development has been studied in many contexts, including in mucociliary tissues. There are 4 Notch receptors in mammalian cells (Notch1-4), which are activated by membrane-bound ligands, members of the Delta and Jagged family, on neighbouring cells. Notch activation leads to a series of cleavage events, culminating in the generation of the Notch intra-cellular domain (NICD), which translocates to the nucleus where it interacts with a transcription factor complex to regulate gene expression. In the epidermis of the Xenopus embryo, activation of Notch suppresses the ciliated cell fate, while inhibition of Notch signaling results in an overproduction of ciliated cells (Deblandre, G.A., et al., Development 126, 4715-4728 (1999)). In the developing mouse airway, expression of the NICD results in an overproduction of secretory cells at the expense of ciliated cells (Guseh, J.S., et al. Development 136, 1751-1759 (2009)), while deletion of Pofut1, an O-fucosyltransferase required for Notch-ligand interactions (Stahl, M., et al., The Journal of biological chemistry 283, 13638-13651 (2008)), or Rbpjk, a core nuclear effector of Notch signalling (Fortini, M.E., Dev Cell 16, 633-647 (2009)), results in an increase in the number of ciliated cells and a near absence of secretory cells (Tsao, P.N., et al., Development 136, 2297-2307 (2009)). A recent study using Notch receptor-specific knockouts suggested that Notch2 is the critical Notch receptor regulating secretory versus ciliated cell fate in the mouse developing airway (Morimoto, M. et al., Development 139, 4365-4373 (2012)). Despite these recent investigations in the mouse developmental field, the role of the Notch receptors in the adult lung is not yet fully understood.

Hence, there is still the need for a greater understanding of the pathways that regulate the function of the human airway epithelium as well as those which define repair and remodelling in both health and disease. Identifying therapeutic targets in these pathways can lead to therapeutics for treating unmet medical needs such as moderate and severe asthma, cystic fibrosis and chronic obstructive pulmonary disease (COPD).

Gamma secretase is an intramembrane protease that cleaves within the membrane-spanning domain of its substrate proteins, including amyloid β-protein precursor (APP) and Notch. The liberation of NICD for example follows cleavage by gamma secretase. Gamma secretase has been a therapeutic target for some time and has been one of the top targets of Alzheimer's disease (AD) (Wolfe, M., J. Neurochem. (2012), 120, (Suppl. 1), 89-98). Thus, a variety of gamma secretase inhibitors have been developed and one of them even advanced into phase III clinical trials, semigacestat.

### Summary of the Invention

It has now surprisingly been found that gamma secretase inhibitors can reduce this goblet cell metaplasia *in vitro* and *in vivo.* The invention therefore provides a gamma secretase inhibitor for use in the treatment and/or prevention of a respiratory disease. In one embodiment of this aspect, the respiratory disease is selected from cystic fibrosis (CF), primary ciliary dyskinesia, chronic bronchitis, chronic obstructive pulmonary disease (COPD), asthma or respiratory tract infections. In another embodiment of this aspect, the invention provides a gamma secretase inhibitor for use in the treatment and/or prevention of a disease mediated by mucus hyper-secretion or the formation of goblet cells.

The invention also provides for the use of a gamma secretase inhibitor in the manufacture of a medicament for the treatment and/or prevention of a respiratory disease. In one embodiment of this aspect, the respiratory disease is selected from cystic fibrosis (CF), primary ciliary dyskinesia, chronic bronchitis, chronic obstructive pulmonary disease (COPD), asthma or respiratory tract infections.

The invention also provides for a method of treatment and/or prevention of a respiratory disease comprising administering a gamma secretase inhibitor to a subject in need thereof. In one embodiment of this aspect, the respiratory disease is selected from cystic fibrosis (CF), primary ciliary dyskinesia, chronic bronchitis, chronic obstructive pulmonary disease (COPD), asthma or respiratory tract infections. In another embodiment of this aspect, the respiratory disease is selected from diseases mediated by mucus hyper-secretion or the formation of goblet cells.

### Brief Description of the Drawings

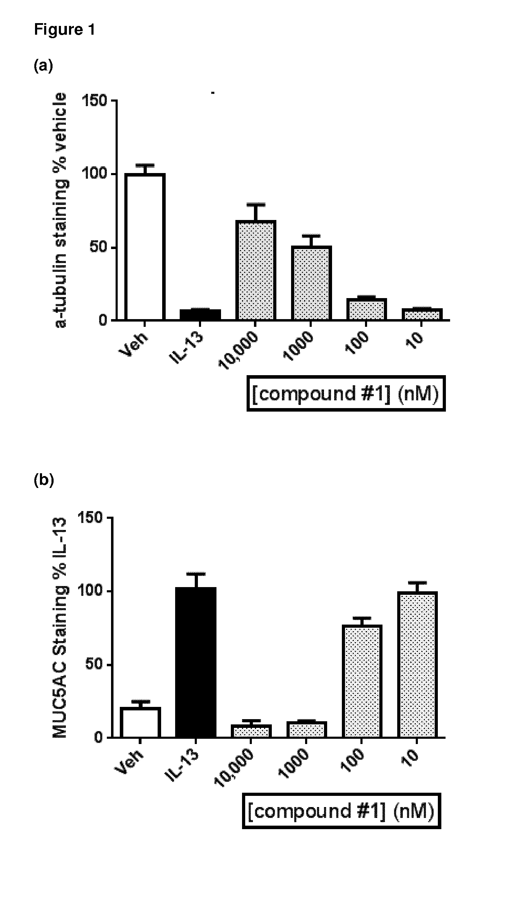

Figure 1: Human airway basal cells were grown on filters at air-liquid interface.
   (a) Effect of compound #1 at 10,000, 1000, 100 and 10nM on IL-13 induced reduction of α-tubulin. Showing relative expression +/- SEM of α-tubulin from 9 replicates over 3 individual studies.
   (b) Effect of compound #1 at 10,000, 1000, 100 and 10nM on IL-13 induced MUC5AC production. Showing relative expression +/- SEM of MUC5AC from 9 replicates over 3 individual studies.

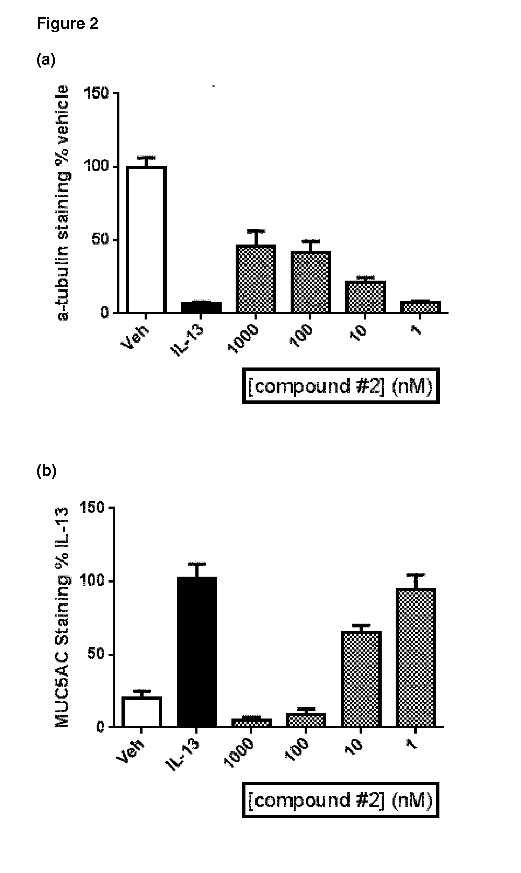

Figure 2: Human airway basal cells were grown on filters at air-liquid interface.
   (a) Effect of compound #2 at 1000, 100, 10 and 1nM on IL-13 induced reduction of α-tubulin. Showing relative expression +/- SEM of α-tubulin from 9 replicates over 3 individual studies.
   (b) Effect of compound #2 at 1000, 100, 10 and 1nM on IL-13 induced MUC5AC production. Showing relative expression of MUC5AC from 9 replicates over 3 individual studies.

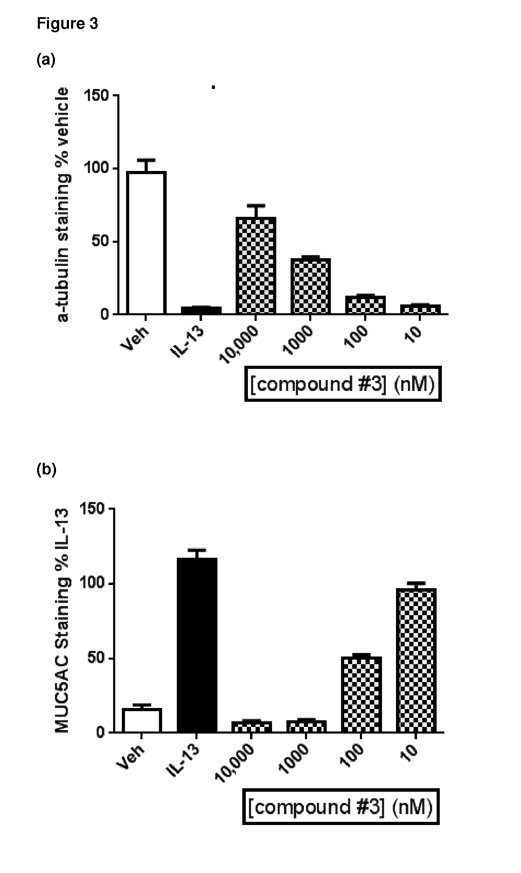

Figure 3: Human airway basal cells were grown on filters at air-liquid interface.
   (a) Effect of compound #3 at 10,000, 1000, 100 and 10nM on IL-13 induced reduction of α-tubulin. Showing relative expression +/- SEM of α-tubulin from 6 replicates over 2 individual studies.
   (b) Effect of compound #3 at 10,000, 1000, 100 and 10nM on IL-13 induced MUC5AC production. Showing relative expression +/- SEM of MUC5AC from 6 replicates over 2 individual studies.

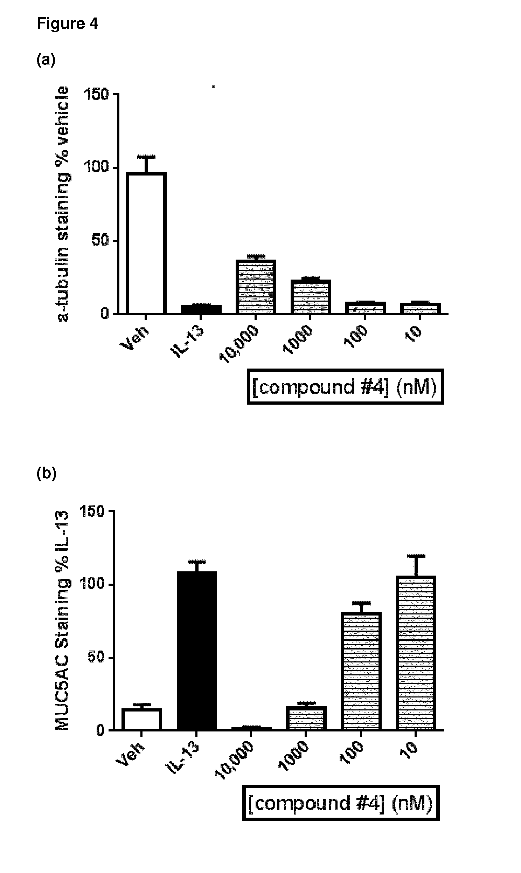

Figure 4: Human airway basal cells were grown on filters at air-liquid interface.
   (a) Effect of compound #4 at 10,000, 1000, 100 and 10nM on IL-13 induced reduction of α-tubulin. Showing relative expression +/- SEM of α-tubulin from 6 replicates over 2 individual studies.
   (b) Effect of compound #4 at 10,000, 1000, 100 and 10nM on IL-13 induced MUC5AC production. Showing relative expression +/- SEM of MUC5AC from 6 replicates over 2 individual studies.

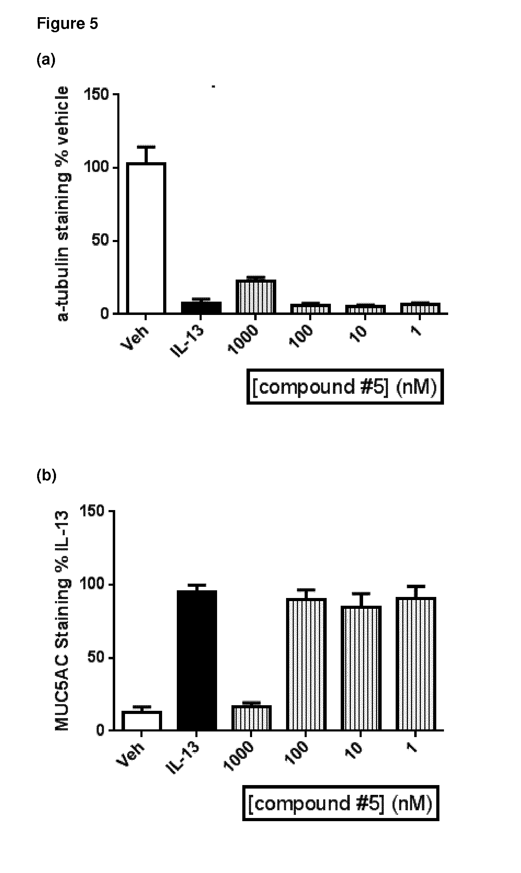

Figure 5: Human airway basal cells were grown on filters at air-liquid interface.
   (a) Effect of compound #5 at 1000, 100, 10 and 1nM on IL-13 induced reduction of α-tubulin. Showing relative expression +/- SEM of α-tubulin from 6 replicates over 2 individual studies.
   (b) Effect of compound #5 at 1000, 100, 10 and 1nM on IL-13 induced MUC5AC production. Showing relative expression +/- SEM of MUC5AC from 6 replicates over 2 individual studies.

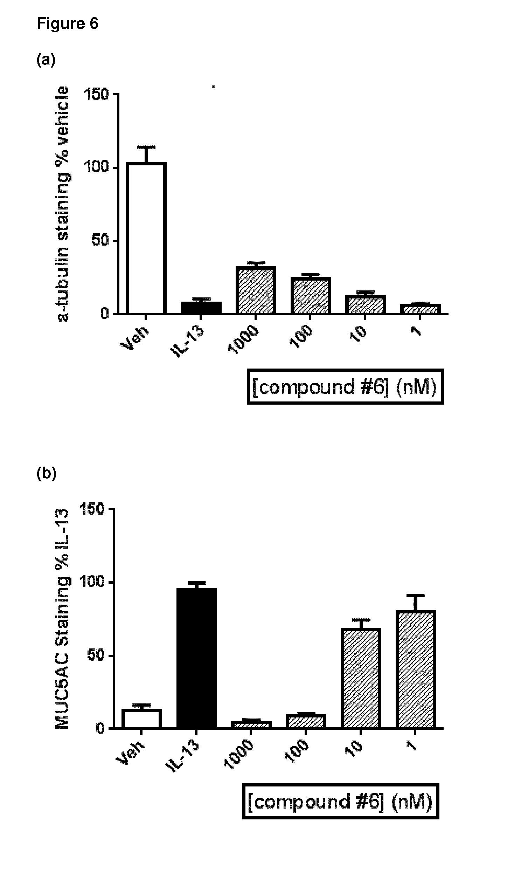

Figure 6: Human airway basal cells were grown on filters at air-liquid interface.
   (a) Effect of compound #6 at 1000, 100, 10 and 1nM on IL-13 induced reduction of α-tubulin. Showing relative expression +/- SEM of α-tubulin from 6 replicates over 2 individual studies.
   (b) Effect of compound #6 at 1000; 100; 10 and 1nM on IL-13 induced MUC5AC production. Showing relative expression +/- SEM of MUC5AC from 6 replicates over 2 individual studies.

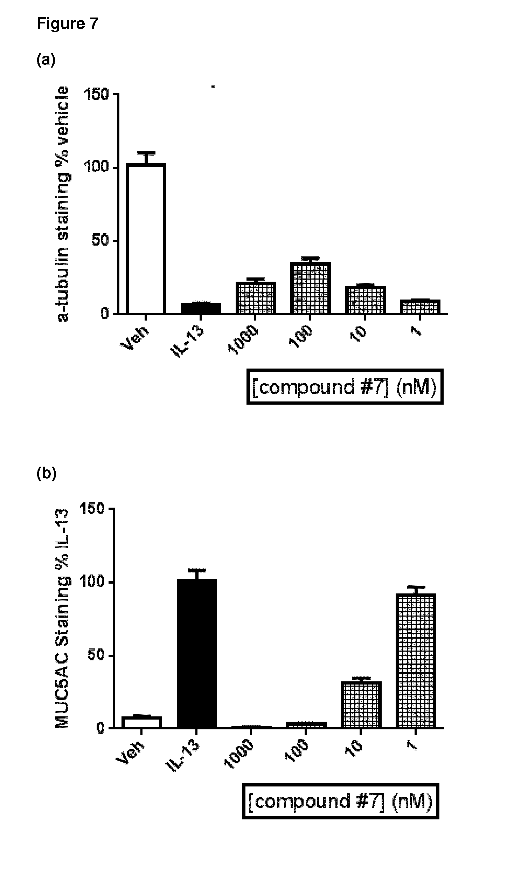

Figure 7: Human airway basal cells were grown on filters at air-liquid interface.
   (a) Effect of compound #7 at 1000, 100, 10 and 1nM on IL-13 induced reduction of α-tubulin. Showing relative expression +/- SEM of α-tubulin from 6 replicates over 2 individual studies.
   (b) Effect of compound #7 at 1000, 100, 10 and 1nM on IL-13 induced MUC5AC production. Showing relative expression +/- SEM of MUC5AC from 6 replicates over 2 individual studies.

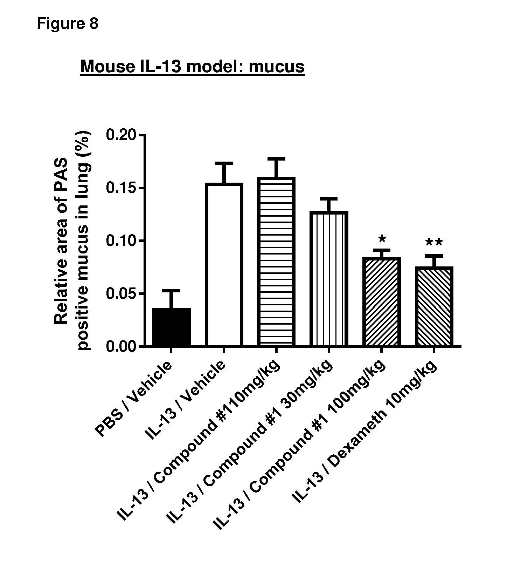

Figure 8: Gamma secretase inhibitor significantly inhibits IL-13 induced increases in lung mucus production *in vivo.*
   Quantification of the relative area of PAS staining in the lung. Effect of compound #1 at 10, 30 and 100 mg/kg. Data are expressed as mean ± SEM. Statistical significance was determined using a parametric one way ANOVA and Dunnetts post-test. GraphPad Prism was used to generate graphs and perform statistical analysis. *p<0.05, **p<0.01, ***p<0.001 denotes statistically significant difference from the IL-13/Vehicle control group.

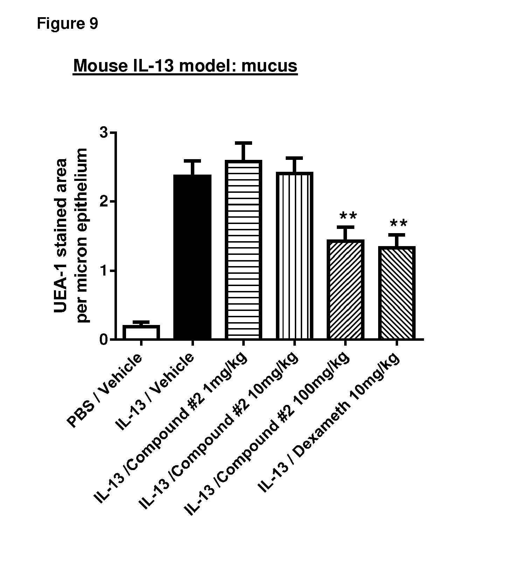

Figure 9: Gamma secretase inhibitor significantly inhibits IL-13 induced increases in lung mucus production *in vivo.*
   Quantification of the relative area of UEA-1 staining in the lung. Effect of compound #2 at 1, 10 and 100 mg/kg. Data are expressed as mean ± SEM. Statistical significance was determined using a parametric one way ANOVA and Dunnetts post-test. GraphPad Prism was used to generate graphs and perform statistical analysis. *p<0.05, **p<0.01, ***p<0.001 denotes statistically significant difference from the IL-13/Vehicle control group.

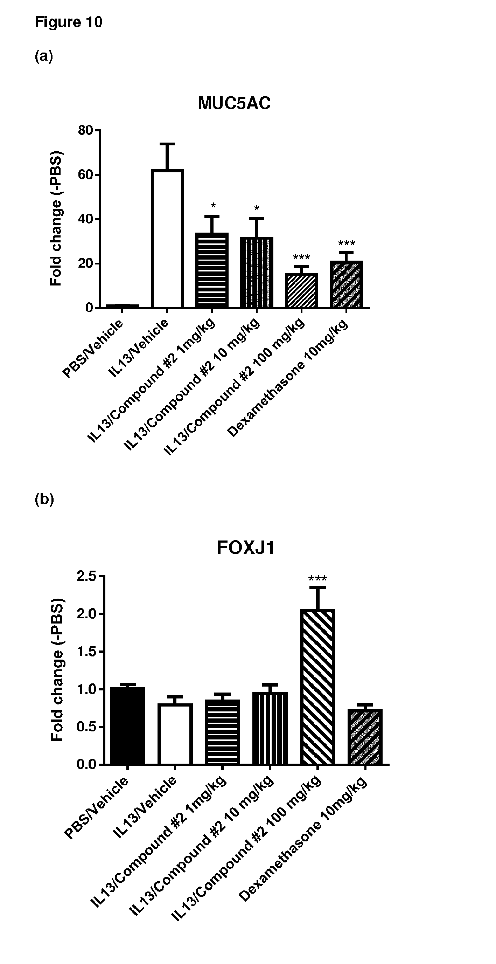

Figure 10: Gamma secretase inhibitor significantly inhibits IL-13 induced increases in lung mucus production *in vivo.*
   Quantitative RT-PCR analysis from RNA isolated from whole lung tissue after treatment with compound #2 (see Figure 9). (a) MUC5AC (goblet) and (b) Foxj1 (ciliated) expression in lungs. Data are expressed as mean ± SEM. Statistical significance was determined using a parametric one way ANOVA and Dunnetts post-test. GraphPad Prism was used to generate graphs and perform statistical analysis. *p<0.05, **p<0.01,
   ***p<0.001 denotes statistically significant difference from the IL-13/Vehicle control group.

### Detailed Description of the Invention

### Definitions

For purposes of interpreting this specification, the following definitions will apply and whenever appropriate, terms used in the singular will also include the plural and vice versa. Additional definitions are set forth throughout the detailed description.

The term "gamma secretase inhibitor" refers to any molecule capable of inhibiting cleaving of Notch and thus the generation of NICD. An overview of gamma secretase inhibitors can be found in Wolfe, M. et al., J. Neurochem. (2012), 120, (Suppl. 1), 89-98. It follows that any forms of "gamma secretase inhibitors" such as salts, co-crystals, crystalline forms, pro-drugs, etc., are included within this term.

As used herein, the term "subject" includes any human or non-human animal.

The term "non-human animal" includes all vertebrates, e.g., mammals and non-mammals, such as non-human primates, sheep, dogs, cats, horses, cows, chickens, amphibians, reptiles, etc.

The term "treat", "treating", "treatment", "prevent", "preventing" or "prevention" includes therapeutic treatments, prophylactic treatments and applications in which one reduces the risk that a subject will develop a disorder or other risk factor. Treatment and/or prevention does not require the complete curing of a disorder and encompasses the reduction of the symptoms or underlying risk factors or at least a slowing down of the progression of the disease.

The term "comprising" means "including" as well as "consisting" e.g., a composition "comprising" X may consist exclusively of X or may include something additional e.g., X + Y.

### Inflammatory cytokine-driven goblet cell metaplasia and mucus hyper-secretion

A number of inflammatory cytokines and growth factors have been reported to increase the expression of MUC5AC in airway epithelial cells (Chen, Y., et al., The Journal of biological chemistry 278, 17036-17043 (2003); Fujisawa, T., et al., J Immunol 183, 6236-6243 (2009); Gray, T., et al., American journal of physiology. Lung cellular and molecular physiology 286, L320-330 (2004) and Kim, Y.D., et al., Molecular pharmacology 62, 1112-1118 (2002)), a mucin that is up-regulated in a number of airway diseases, including asthma and COPD (Fahy, J.V. & Dickey, B.F, The New England journal of medicine 363, 2233-2247 (2010)). However, the mechanism by which soluble factors lead to increased MUC5AC levels is not fully understood.

IL-13 has been shown to be a key mediator of allergic asthma in numerous pre-clinical and clinical studies. It is known that polymorphisms in both IL-13 and the IL-4 receptor (a component of the IL-13 receptor complex) are associated with asthma susceptibility and that IL-13 induced pathways are linked to airway responses in asthma (reviewed in: Ingram & Kraft, 2012; J All Clin Imm 130(4):829; Wills-Karp, M., Immunological reviews 202, 175-190 (2004)). Amongst the reported effects of IL-13 that are of relevance to asthma, several groups have demonstrated that IL-13 acts directly on the epithelium to drive a goblet cell metaplasia phenotype by increasing the number of goblet cells *in vitro* and *in vivo* by altering the airway epithelial cell fate (Atherton, H.C. et al., American journal of physiology. Lung cellular and molecular physiology 285, L730-739 (2003); Kuperman, D.A., et al., Nature medicine 8, 885-889 (2002) and Laoukili, J., et al., The Journal of clinical investigation 108, 1817-1824 (2001)).

The balance and distribution of epithelial cell types is required to maintain tissue homeostasis and perturbations of this balance are hallmarks of human respiratory diseases, including goblet cell metaplasia and enhanced mucus secretion in asthma and COPD. In the conducting airway, basal cells act as progenitors for both secretory and ciliated cells. Studying the development and function of these varied airway cell types and to identify mechanism regulating cell fate decisions in the airways has been advanced in the recent years by air-liquid interface (ALI) cultures of primary epithelia (Gray, T.E. et al, American journal of respiratory cell and molecular biology 14, 104-112 (1996)). In these systems, cells are cultured on a permeable support to confluency, at which time media is removed from the apical side resulting in polarization and differentiation of the epithelium. With respect to the human proximal airways, ALI cultures of primary tracheo-bronchial cells replicate the architecture of the native epithelium, together with ion transport function and an intact mucociliary clearance system.

Using ALI cultures and an *in vivo* IL-13 mouse model, we are able to show that IL-13 stimulation results in goblet cell differentiation with increased MUC5AC levels and reduced numbers of ciliated cells with decreased α-tubulin levels. Thus, IL-13 stimulation drives away from an α-tubulin phenotype towards a goblet cell phenotype. We show herein that this IL-13 induced goblet cell formation can be inhibited via treatment with a gamma secretase inhibitor. Thus, our data supports a gamma secretase inhibitor for use in the treatment of respiratory diseases such as cystic fibrosis (CF), primary ciliary dyskinesia, chronic bronchitis, chronic obstructive pulmonary disease (COPD), asthma or respiratory tract infections, characterized by excessive goblet cell formation and mucus hyper-secretion, regardless of the disease stimulus.

### Embodiments of the invention

Various (enumerated) embodiments of the invention are described herein. It will be recognised that features specified in each embodiment may be combined with other specified features to provide further embodiments of the present invention.

Embodiment 1: A gamma secretase inhibitor for use in the treatment and/or prevention of respiratory diseases.

Embodiment 2: A gamma secretase inhibitor for use in the treatment and/or prevention of respiratory diseases, wherein the gamma secretase inhibitor inhibits goblet cell formation and/or mucus hyper-secretion.

Embodiment 3: The gamma secretase inhibitor for use in the treatment and/or prevention of respiratory diseases according to embodiment 1 or 2, wherein the respiratory disease is selected from cystic fibrosis (CF), primary ciliary dyskinesia, chronic bronchitis, chronic obstructive pulmonary disease (COPD), asthma or respiratory tract infections.

Embodiment 4: The gamma secretase inhibitor for use in the treatment and/or prevention of respiratory diseases according to any one of embodiments 1 to 3, wherein the respiratory disease is an IL-13-mediated respiratory disease.

Embodiment 5. The gamma secretase inhibitor for use in the treatment and/or prevention of respiratory diseases according to any one of embodiments 1 to 4, wherein the gamma secretase inhibitor is a non-peptide compound.

Embodiment 6: The gamma secretase inhibitor for use in the treatment and/or prevention of respiratory diseases according to any one of embodiments 1 to 5, wherein the gamma secretase inhibitor is selected from any one of compounds #1 to #7 as defined herein.

Embodiment 7: The gamma secretase inhibitor for use in the treatment and/or prevention of respiratory diseases according to any one of embodiments 1 to 6, wherein the respiratory disease is cystic fibrosis (CF).

Embodiment 8: The gamma secretase inhibitor for use in the treatment and/or prevention of respiratory diseases according to any one of embodiments 1 to 6, wherein the respiratory disease is chronic obstructive pulmonary disease (COPD).

Embodiment 9: The gamma secretase inhibitor for use in the treatment and/or prevention of respiratory diseases according to any one of embodiments 1 to 6, wherein the respiratory disease is asthma.

Embodiment 10: The gamma secretase inhibitor for use in the treatment and/or prevention of respiratory diseases according to any one of embodiments 1 to 6, wherein the respiratory disease is chronic bronchitis.

Embodiment 11: The gamma secretase inhibitor for use in the treatment and/or prevention of respiratory diseases according to any one of embodiments 1 to 6, wherein the respiratory disease is respiratory tract infection.

Embodiment 12: The gamma secretase inhibitor for use in the treatment and/or prevention of respiratory diseases according to any one of embodiments 1 to 6, wherein the respiratory disease is primary ciliary dyskinesia.

Embodiment 13: Use of a gamma secretase inhibitor according to any one of embodiments 1 to 12 in the manufacture of a medicament for the treatment and/or prevention of respiratory diseases.

Embodiment 14: A method of treatment and/or prevention of respiratory diseases comprising administering a gamma secretase inhibitor according to any one of embodiments 1 to 12 to a subject in need thereof.

Embodiment 15: Use of a gamma secretase inhibitor according to any one of embodiments 1 to 12 for the treatment and/or prevention of respiratory diseases.

### Pharmaceutical compositions

The invention provides for a gamma secretase inhibitor formulated with a pharmaceutical acceptable carrier for use in the treatment and/or prevention of respiratory diseases. Pharmaceutical compositions used in the invention also can be administered in combination therapy, i.e., combined with other agents. For example, the combination therapy can include a gamma secretase inhibitor administered concomitantly to antibiotics, steroids agents, mucolytics, bronchodilators, CFTR activity modulators, ENaC blockers; TMEM activators/potentiators; CFTR correctors; and CFTR potentiators. Suitable ENaC blockers include those disclosed in WO2009/074575 and WO2012/035158. Suitable CFTR correctors include those disclosed in WO2007/056341, WO2010/053471, WO2010/054138 and WO2012/027247. Suitable CFTR potentiators include those disclosed in WO2006/002421, WO2011/113894 and US8247436. Such combinations may be administered simultaneously or sequentially. If administered sequentially, the period between administration of each agent may be a week or less, (e.g. a day or less, 12 hours or less, 6 hours or less, 1 hour or less, 30 minutes or less). The compositions are preferably formulated at physiological pH.

### Materials and methodology

Gamma secretase inhibitors:

### Compound #1:

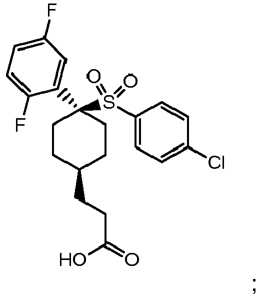

3-((1s,4r)-4-((4-chlorophenyl)sulfonyl)-4-(2,5-difluorophenyl)cyclohexyl)propanoic acid; compound #1 is commercially available or may be prepared according to known methods, for example as described in Scott et al., Journal of Organic Chemistry (2007), 72(11), 4149-4155;

### Compound #2:

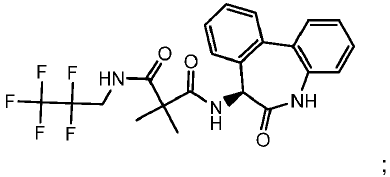

(S)-2,2-dimethyl-N1-(6-oxo-6,7-dihydro-5H-dibenzo[b,d]azepin-7-yl)-N3-(2,2,3,3,3-pentafluoropropyl)malonamide; compound #2 is commercially available or may be prepared according to known methods, for example as described in US 20050054633 A1 (Flohr et al.);

### Compound #3:

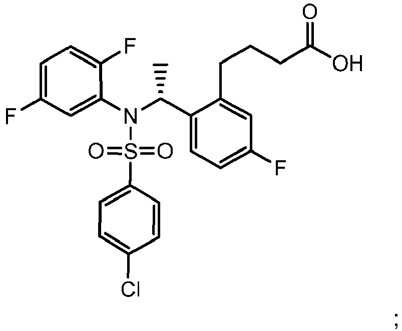

(R)-4-(2-(1-(4-chloro-N-(2,5-difluorophenyl)phenylsulfonamido)ethyl)-5-fluorophenyl)butanoic acid; BMS299897; compound #3 is commercially available (e.g. from Diverchem SA) or may be prepared according to known methods, for example as described in WO2000050391 A1 (Merck, Bristol-Myers Squibb);

### Compound #4:

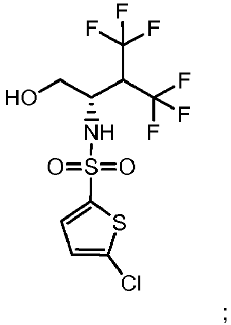

(S)-5-chloro-N-(4,4,4-trifluoro-1-hydroxy-3-(trifluoromethyl)butan-2-yl)thiophene-2-sulfonamide; begacestat; compound #4 is commercially available (e.g. from Pharmeron Inc.) or may be prepared according to known methods, for example as described in Mayer et al., Journal of Medicinal Chemistry (2008), 51 (23), 7348-7351;

### Compound #5:

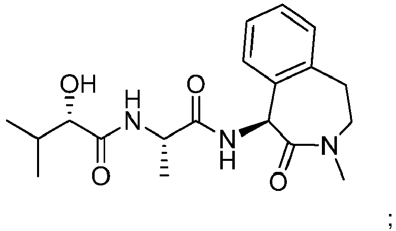

(S)-2-hydroxy-3-methyl-N-((S)-1-(((S)-3-methyl-2-oxo-2,3,4,5-tetrahydro-1H-benzo[d]azepin-1-yl)amino)-1-oxopropan-2-yl)butanamide; semagacestat; compound #5 is commercially available or may be prepared according to known methods, for example as described in Boini et al., Synthesis (2009), (12), 1983-1986;

### Compound #6:

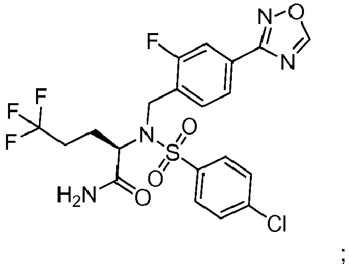

(R)-2-(4-chloro-N-(2-fluoro-4-(1,2,4-oxadiazol-3-yl)benzyl)phenylsulfonamido)-5,5,5-trifluoropentanamide; avagacestat; compound #6 is commercially available (e.g. from Pharmeron Inc.) or may be prepared according to known methods, for example as described in Maharvi, Tetrahedron Letters (2010), 51 (50), 6542-6544;

### Compound #7:

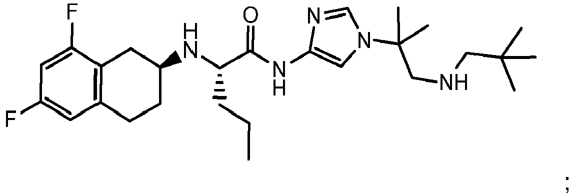

(S)-2-(((S)-6,8-difluoro-1,2,3,4-tetrahydronaphthalen-2-yl)amino)-N-(1-(2-methyl-1-(neopentylamino)propan-2-yl)-1 H-imidazol-4-yl)pentanamide; PF-0308014; compound #7 is commercially available or may be prepared according to known methods, for example as described in Brodney et al., Bioorganic & Medicinal Chemistry Letters (2011), 21(9), 2637-2640;

### (A) Gamma secretase inhibition on IL-13 treated human bronchial epithelial cells (HBECs)

### Human Tissue Culture

Human bronchial epithelial cells (HBECs; Lonza)were seeded in T162 flasks and were grown in growth media (Lonza Bronchial epithelial cell basal medium (Lonza) plus Bronchial epithelial cell growth medium singlequots (Lonza), which are supplemented with bovine pituitary extract, hydrocortisone, human epidermal growth factor, epinephrine, transferrin, insulin, retinoic acid, triiodothyronine, gentamicin and amphotocerin B). At around 80% confluent, cells were detached using Trypsin (Invitrogen). Trypsin was neutralized using Trypsin neutralizing solution (Lonza), cells centrifuged at 1200rpm for 5mins and the pellet re-suspended in differentiation media (50% bronchial epithelial cell basal medium (Lonza), 50% Dulbecco's Modified Eagle Medium (w/o sodium pyruvate with 4500mg/L glucose; Invitrogen), 50nM Trans Retinoic acid (Sigma) and Bronchial epithelial cell growth medium singlequots (Lonza; as above but without triiodothyronine or retinoic acid).

The cells were counted and seeded onto 12 well inserts (Transwell plates, Costar) at 8.25x10⁴ cells/insert in differentiation media. The cells were cultured on the inserts submerged in media (1ml basolateral, 0.5ml apical) until confluent (approximately 7 days) before being taken to air-liquid interface (ALI). At ALI transition the apical media is removed and the cells are fed and treated basolaterally with media.

Treatment of HBECs commenced at transition to ALI and cells were treated basolaterally with cytokine IL-13 (1ng/ml, Peprotech) or vehicle (0.1% DMSO) in the presence or absence of gamma secretase inhibitors (compounds #1-7; concentrations ranging between 1 nM - 1000nM) for a duration of 2 weeks. The medium containing the treatments was refreshed every 48-72hrs. In addition, the apical surface of the cells was washed twice weekly with warm phosphate buffered saline (Invitrogen). During the 2 weeks treatment time, the cells differentiated into a population comprising ciliated cells and mucus secreting goblet cells.

### Microscopy

Air-liquid interface (ALI) cultures were processed for immunofluorescence analysis after 14 days of culture at ALI by rinsing the apical surface of each filter with PBS, and then fixing in 4% paraformaldehyde (500ul applied to apical followed by 1ml basolateral application) for 4 hours. Filters were washed with IF buffer (130 mM NaCl, 7 mM Na₂HPO₄, 3.5 mM NaH₂PO₄, 7.7 mM NaN₃, 0.1% bovine serum albumin, 0.2% Triton X-100, 0.05% Tween-20), blocked with IF wash containing 10% goat serum, and stained with primary antibody (MUC5AC (clone 45M1; Thermo Scientific) or acetylated α-tubulin (clone 6-11 B-1; Sigma-Aldrich) at a 1:200 dilution) in IF wash containing 10% goat serum overnight at 4 °C. Secondary antibodies (Alexa Fluor 488 and 568; Invitrogen) were used at a 1:200 dilution in IF buffer containing 10% goat serum. To quantify the total staining area of ALI cultures, 10 x 13 images were collected with a plan Neofluar 10× 0.3 NA Ph1 objective (EC; Carl Zeiss, Inc.) on a microscope (Axiovert 200; Carl Zeiss, Inc.) equipped with a motorized stage and a camera (Orca-ER-1394; Hamamatsu Photonics) controlled by Axiovision software (Carl Zeiss, Inc.) and used to generate a single composite image using the MOSAIX function in Zeiss Axiovision. Quantification of the total staining area for α-tubulin and MUC5AC was performed with ImageJ.

### (B) Effect of gamma secretase inhibitors in an in vivo IL-13 mouse model

Female Balb/c mice (20-25 g) were obtained from Charles River (Morgate, UK). Mice were housed under specific pathogen free conditions and were provided with food and water ad libitum. Experiments were performed in accordance with the UK Animals Scientific Procedures Act 1986.

Mice received 0.5 µg of recombinant mouse IL-13 (Ebiosciences, UK) or phosphate-buffered saline (PBS) intranasally on 3 consecutive days (days 1 - 3). Mice received either vehicle (0.5% methylcellulose 0.5% Tween80) or compound #1 (10 / 30 / 100mg/kg) or compound #2 (1 / 10 / 100mg/kg) orally BID on days 1-3. An additional group of mice received dexamethasone (10mg/kg) orally as a positive control.

Mice were euthanized 24 hours after their final PBS or IL-13 administration. The left lungs were excised, inflated with 10% neutral buffered formalin (NBF) and preserved in NBF. Lungs were embedded in paraffin wax and lung sections obtained for each animal. Sections were stained with Periodic acid-Schiff (PAS) stain or Ulex-europaeus agglutinin-1 (UEA-1) stain for mucus.

PAS positive staining was quantified in lung sections using Definiens Image Analysis software. Two distinct lung sections were analyzed per mouse and the total area of PAS positive staining within the sections was determined. The PAS positive staining was normalized to the area of tissue analyzed and represented as 'Relative Area PAS Positive Mucus (%)'.

The area of UEA-1 staining was assessed under a Zeiss Axioplan microscope (X10 magnification) with an imaging associates KS400 image analyser. UEA-1-positive stained area was assessed in the large left lung lobe, which had been sliced into four sections. Up to 14 fields in the lung were scored for each animal. Data are represented as 'UEA-1 stained area per micron epithelium', which is defined as the ratio of stained area to the length of epithelium scored.

Data are expressed as mean ± SEM. Statistical significance was determined using a parametric one way ANOVA and Dunnetts post-test. GraphPad Prism was used to generate graphs and perform statistical analysis. *p<0.05, **p<0.01, ***p<0.001 denotes statistically significant difference from the IL-13/Vehicle control group.

### RNA isolation and quantitative PCR

To isolate RNA from mouse tissue, approximately 40 mg of tissue was placed in a 2ml tube containing hard ceramic beads (Precellys lysing kit cat number 03961-1-002) and 1.4 ml of TRK Lysis buffer(E.Z.N.A.^{®} Total RNA Midiprep Kit, cat number: R6664-02). Tissue was sheared using a Precellis 24 tissue homogenizer. Once homogenization completed, samples were centrifuged for 10min at 3000G. Supernatant was collected in a 15ml falcon tube containing 600ul of TRK Lysis buffer (final volume of 2ml). RNA from the sheared tissue was purified using E.Z.N.A.^{®} Total RNA Midiprep Kit, using manufacturer's specifications.TaqMan Reverse Transcription Reagents (Invitrogen) were used to generate cDNA from 500ng of total RNA. Quantitative PCR was performed on 7500 Fast Real Time PCR System (Applied Biosystems), using 50ng of cDNA per reaction with the following Taqman probes (Applied Biosystems): FOXJ1; Mm01267279_m1 and Muc5ac; Mm01276718_m1.

### Results

### Gamma secretase inhibitors significantly inhibit IL-13 induced increases in lung mucus production in vitro

The gamma secretase inhibitors compound #1 to #7 have all shown to dose dependently decrease the IL-13 induced MUC5AC production in HBEC cells (ALI) back to or below baseline levels (see Figures 1-7(b)). Furthermore, an increase in α-tubulin expression can also be observed in a dose dependent fashion (see Figures 1-7(a)). MUC5AC and α-tubulin are markers for goblet cells and ciliated cells, respectively. IL-13 stimulation results in goblet cell differentiation with increased MUC5AC levels; and reduced numbers of ciliated cells with decreased α-tubulin levels. Thus, IL-13 stimulation drives away from an α-tubulin phenotype towards a goblet cell phenotype. The results disclosed herein demonstrate that gamma secretase inhibitors can inhibit the IL-13 induced promotion of goblet cell differentiation (as shown by the decrease of MUC5AC) and also suggests a partial restoration of ciliated cells (as shown by the increase in α-tubulin).

### Gamma secretase inhibitors significantly inhibit IL-13 induced increases in lung mucus production in an in vivo mouse model.

Mucus staining of mouse lung sections revealed that IL-13 treatment drove a goblet cell metaplasia phenotype in vivo. Treatment with compound #1 or compound #2 (100mg/kg p.o.) significantly inhibited IL-13 driven goblet cell metaplasia in vivo, as evidenced by reduced mucus staining of lung sections (see Figures 8 and 9). In the study with compound #2, we verified this observation using quantitative RT-PCR analysis of MUC5AC levels from RNA isolated from whole lung tissue and also found that treatment with the gamma secretase inhibitor compound #2 resulted in a concomitant increase in the ciliated cell marker Foxj1.

## Claims

1. A gamma secretase inhibitor for use in the treatment and/or prevention of respiratory diseases.

2. A gamma secretase inhibitor for use in the treatment and/or prevention of respiratory diseases, wherein the gamma secretase inhibitor inhibits goblet cell formation and/or mucus hyper-secretion.

3. The gamma secretase inhibitor for use in the treatment and/or prevention of respiratory diseases according to embodiment 1 or 2, wherein the respiratory disease is selected from cystic fibrosis (CF), primary ciliary dyskinesia, chronic bronchitis, chronic obstructive pulmonary disease (COPD), asthma or respiratory tract infections.

4. The gamma secretase inhibitor for use in the treatment and/or prevention of respiratory diseases according to any one of embodiments 1 to 3, wherein the respiratory disease is an IL-13-mediated respiratory disease.

5. The gamma secretase inhibitor for use in the treatment and/or prevention of respiratory diseases according to any one of embodiments 1 to 4, wherein the gamma secretase inhibitor is a non-peptide compound.

6. : The gamma secretase inhibitor for use in the treatment and/or prevention of respiratory diseases according to any one of embodiments 1 to 5, wherein the gamma secretase inhibitor is selected from any one of compounds #1 to #7 as defined herein.

7. The gamma secretase inhibitor for use in the treatment and/or prevention of respiratory diseases according to any one of embodiments 1 to 6, wherein the respiratory disease is cystic fibrosis (CF).

8. The gamma secretase inhibitor for use in the treatment and/or prevention of respiratory diseases according to any one of embodiments 1 to 6, wherein the respiratory disease is chronic obstructive pulmonary disease (COPD).

9. The gamma secretase inhibitor for use in the treatment and/or prevention of respiratory diseases according to any one of embodiments 1 to 6, wherein the respiratory disease is asthma.

10. The gamma secretase inhibitor for use in the treatment and/or prevention of respiratory diseases according to any one of embodiments 1 to 6, wherein the respiratory disease is chronic bronchitis.

11. The gamma secretase inhibitor for use in the treatment and/or prevention of respiratory diseases according to any one of embodiments 1 to 6, wherein the respiratory disease is respiratory tract infection.

12. The gamma secretase inhibitor for use in the treatment and/or prevention of respiratory diseases according to any one of embodiments 1 to 6, wherein the respiratory disease is primary ciliary dyskinesia.

13. Use of a gamma secretase inhibitor according to any one of embodiments 1 to 12 in the manufacture of a medicament for the treatment and/or prevention of respiratory diseases.

14. A method of treatment and/or prevention of respiratory diseases comprising administering a gamma secretase inhibitor according to any one of embodiments 1 to 12 to a subject in need thereof.

15. Use of a gamma secretase inhibitor according to any one of embodiments 1 to 12 for the treatment and/or prevention of respiratory diseases.
